# EUROPEAN PATENT APPLICATION

(11) **EP 2 077 336 A1**
(43) Date of publication of application: **08.07.2009**
(21) Application number: 07123578.2
(22) Date of filing: 19.12.2007
(51) Int. Cl.: C12Q 1/68

(54) **Device and method for parallel quantitative analysis of multiple nucleic acids**

(71) Applicant: Koninklijke Philips Electronics N.V., 5621 BA Eindhoven (NL)
(72) Inventor: The designation of the inventor has not yet been filed
(74) Representative: Van Velzen, Maaike Mathilde

(57) **Abstract**

The present invention relates to a process for conducting real-time PCR, and to a device for conducting the method of the present invention. The invention is especially suited for the simultaneous identification and quantification of nucleic acids present in a sample, e.g. a biological sample.

Further, this invention describes a method for simultaneous quantitative analysis of multiple nucleic acid sequences in a single compartment by using an integrated nucleic acid microarray combined with a highly surface-specific readout device.

The invention relates to a device wherein a surface which is either part of the chamber surface or a surface that is created in the reaction chamber, such as bead surface, is coated with capture probes and in the same chamber, a PCR reaction takes place.

## Description

### FIELD OF THE INVENTION

The present invention relates to a process for conducting real-time PCR, and to a device for conducting the method of the present invention. The invention is especially suited for the simultaneous identification and quantification of nucleic acids present in a sample, e.g. a biological sample.

Further, this invention relates to a method for simultaneous quantitative analysis of multiple nucleic acid sequences in a single compartment by using e.g. an integrated nucleic acid microarray combined with a highly surface-specific readout device.

The invention relates to a device wherein a surface which is either part of the chamber surface or a surface that is created in the reaction chamber, such as a bead surface, is coated with capture probes and in the same chamber, a PCR reaction takes place.

### BACKGROUND OF THE INVENTION

Polymerase chain reaction (PCR) is a method for amplification of specific nucleic acid sequences. PCR is an enzymatic reaction that takes place in solution. When the amplification process is monitored in Real-time, PCR can be used for quantitative analysis. Real-time PCR normally uses fluorescent reporters, such as intercalating dyes, Taqman probes, Scorpion primers, molecular beacons. When more than one nucleic acid target sequence is to be analyzed, two approaches can be taken. The first approach is to parallelize the reactions, i.e. to run each reaction in a separate compartment. The second approach is to multiplex the reactions, i.e. to run the reactions in the same compartment and to use different fluorophore reporters for each reaction. This approach is limited by the number of fluorophores that can efficiently be discriminated. The current state-of-the-art is that six reactions can be multiplexed.

### SUMMARY OF THE INVENTION

Subject of the present invention is a method for simultaneous real-time quantitative detection of multiple target nucleic acid sequences during amplification wherein the number of simultaneously and quantitatively detected target nucleic acids that are amplified and detected in a single compartment is greater than six, preferably greater than seven, more preferably greater than ten.

Subject of the present invention is a method for simultaneous real-time quantitative detection of multiple target nucleic acid sequences during amplification,
wherein surface-immobilized oligonucleotide probes complementary to said multiple nucleic acid sequences act as capture probes for said multiple amplified nucleic acid sequences,
and wherein detection of said multiple amplified nucleic acid sequences captured at the capture sites is performed with a surface-specific detection method detecting a signal in proximity to the surface.

According to the present invention surface-specific detection is obtained either by a surface specific detection system and/or by surface-specific generation of the signal to be detected.

One embodiment of the invention is a method for simultaneous quantitative detection of multiple target nucleic acid sequences during amplification according to the present invention, wherein primers are labeled and the hybridized labeled amplicons are detected with a surface-specific detection system that detects those labels which are bound to the surface but does essentially not detect labels which are in solution.

Another embodiment of the invention is a method for simultaneous quantitative detection of multiple target nucleic acid sequences according to the present invention, wherein the capture probes are probes with a fluorescent label and a quencher in close proximity due to the structure of the probes, and a signal may be detected in case an amplicon hybridizes to said capture probes.

Another particular embodiment of the present invention is a method for simultaneous quantitative detection of multiple target nucleic acid sequences, wherein the capture probes are probes with a fluorescent label and a quencher and a signal may be detected in case a target nucleic acid sequence hybridizes to said capture probes causing enzymatic hydrolysis of the capture probe thereby liberating said quencher from said fluorophore.

Yet another embodiment of the invention is a method for simultaneous quantitative detection of multiple target nucleic acid sequences according to the present invention, wherein the capture probes are immobilized on individually identifiable beads.

Further, subject of the present invention is a device for conducting a method according to the present invention comprising a compartment of which an inner surface is coated with capture probes for multiple target nucleic acid sequences and a surface-specific detection system that detects those labels which are bound to the surface but does essentially not detect labels which are in solution.

Another embodiment of the invention is a device for conducting a method according to the present invention comprising a compartment of which an inner surface is coated with capture probes for multiple target nucleic acid sequences and wherein the capture probes are labeled and a signal may be detected in case an amplicon hybridizes to said capture probes.

Another embodiment of the invention is a device for conducting a method according to the present invention, wherein the capture probes are probes with a fluorescent label and a quencher, and a signal may be detected in case a target nucleic acid sequence hybridized to said capture probes causing enzymatic hydrolysis of the capture probes thereby liberating said quencher from said fluorophore.

Yet another embodiment of the invention is a device for conducting a method according to the present invention comprising a compartment with individually identifiable beads wherein capture probes for multiple target nucleic acid sequences are immobilized on said individually identifiable beads.

### BRIEF DESCRIPTION OF THE DRAWINGS

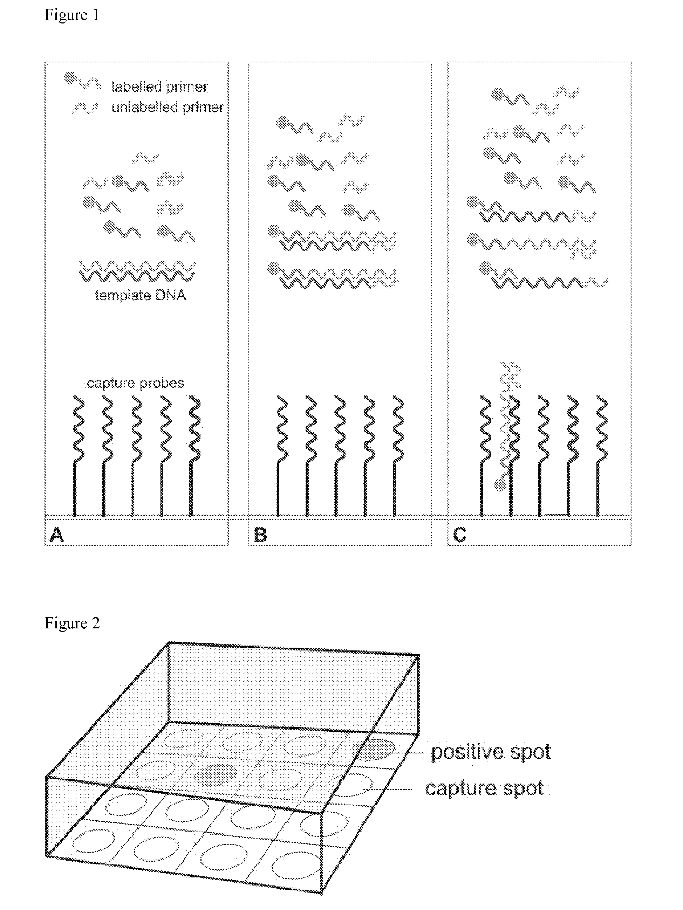

Fig. 1: (A) A container with capture probes (DNA oligonucleotides) is filled with a mixture containing target template DNA, PCR primers (1 of which is labeled) and PCR master mix. (B) As one of the primers contains a fluorophore, amplicons that are generated during PCR thermocycling will be fluorescently labeled. (C) The sequence of the capture probes is complementary to the sequence of the labeled strand of the amplicon. Amplicons are thus capable of hybridizing to the capture probes during the annealing stage of a thermocycle.
Fig. 2: Schematic representation of an array of capture spots that can monitor the amplification of multiple different amplicons in the same fluid. The space above the array is filled with a homogeneous PCR mixture. The use of an array allows for high PCR multiplexing grades

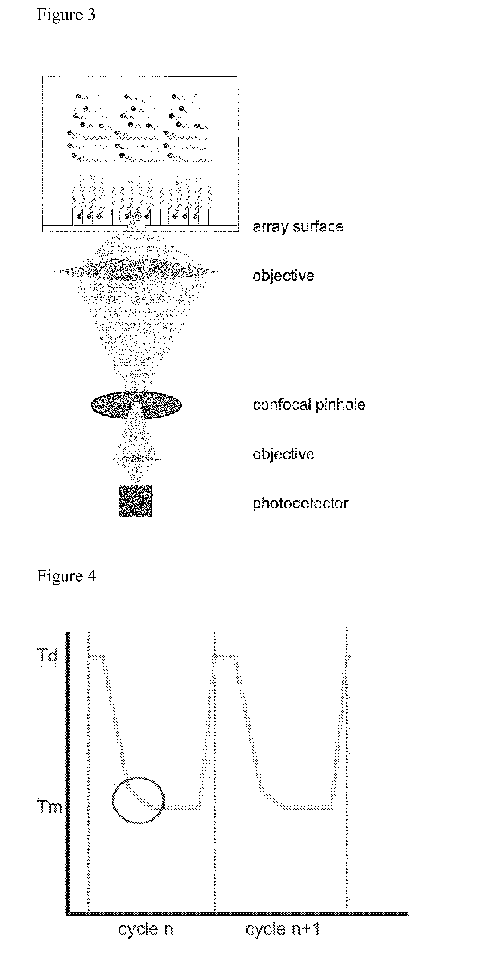

Fig. 3: Schematic representation of a confocal reader that measures the amount of fluorescent molecules hybridized to the capture probes. Although the fluid above the capture surface is loaded with fluorophores, only the fluorophores close to the capture surface are detected
Fig. 4: A schematic representation of a PCR thermocycle with a non-uniform cooling rate. A 2-step cycle (Td= temperature of denaturation; Tm= temperature of annealing) is shown (a 3-step cyle with an additional hold at the elongation temperature is also possible). The last part of the cooling stage from Td to Tm can be done at a lower rate (indicated by blue circle). This allows amplified strands to hybridize to the capture probes before primer annealing and elongations have taken place when capture probes with a melting temperature above the melting temperature of the primers are used.

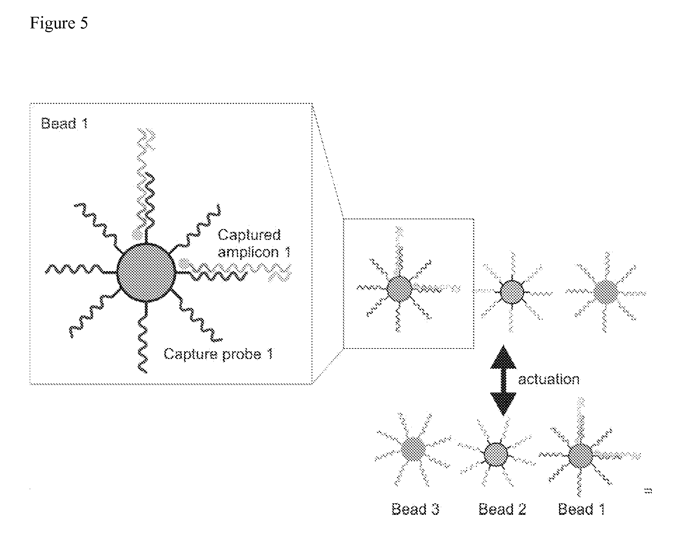

Fig. 5: Schematic representation of a bead array. Besides primers, template DNA, enzyme and dNTPs (not shown), the reaction mixture also contains beads coated with capture probes. In this figure and example with 3 different capture beads is shown. Capture bead 1 is coated with capture probe 1 and contains an individually identifiable code 1 (preferentially a color code, but other codes, e.g. size-, radio- or bar-codes are also possible). Capture probe 1 can capture amplicon 1, because it has a sequence complementary to part of this amplicon. Similarly, capture bead n contains individually identifiable code n and is coated with capture probe n which is complementary to part of amplicon n. All the capture beads are allowed to be freely dispersed in the reaction fluid to increase the rate of amplicon capture. Subsequently, the beads can be brought to the surface (e.g. by magnetic actuation in the case of (para)magnetic beads or by dielectorphoresis in the case of non-magnetic beads) where each bead can be identified and the amount of captured amplicon can be quantified.

### DETAILED DESCRIPTION OF EMBODIMENTS

Subject of the present invention is a method for simultaneous real-time quantitative detection of multiple target nucleic acid sequences during amplification wherein the number of simultaneously and quantitatively detected target nucleic acids that are amplified and detected in a single compartment is greater than six, preferably greater than seven, more preferably greater than ten.

Amplification may be performed by various enzymatic methods including PCR (polymerase chain reaction), NASBA (nucleic acid sequence based amplification), TMA (transcription mediated amplification), and rolling circle amplification. Enzymatic amplification methods suitable for the present invention are known to a person skilled in the art.

Subject of the present invention is a method for simultaneous real-time quantitative detection of multiple target nucleic acid sequences during amplification,
wherein surface-immobilized oligonucleotide probes complementary to said multiple nucleic acid sequences act as capture probes for said multiple amplified nucleic acid sequences,
and wherein detection of said multiple amplified nucleic acid sequences captured to the captured sites is performed with a surface-specific detection method detecting a signal in proximity to the surface.

According to the present invention surface specific detection is obtained either by a surface specific detection system and/or by surface-specific generation of the signal to be detected.

The inventive method for simultaneous quantitative detection of multiple target nucleic acid sequences during amplification according to the present invention may comprise the following steps:
- providing a device having a reaction compartment, wherein the reaction compartment comprises a surface that is coated with multiple capture probes for said multiple nucleic acid sequences,
- adding multiple target nucleic acids and an amplification mixture comprising amplification primers for the amplification of said multiple target nucleic acids to the compartment,
- starting amplification thermocycling during which specific segments of said target nucleic acids are amplified thereby creating amplicons,
wherein during the annealing phase of the amplification reaction a portion of the amplicons hybridize to the capture probes, and
wherein amplicons which are hybridized to said capture probes are specifically and quantitatively detected, wherein the respective signal which is detected is indicative of the initial respective target nucleic acid concentration and thereby the multiple target nucleic acid sequences are quantitatively detected.

The following definitions apply to this and the other embodiments of the invention:
Surface specific detection means that the contribution to the detected signal by amplicons that are not captured (e.g., floating in the fluid on top of the surface with capture probes) is substantially suppressed, which means preferably suppressed by at least a factor 50, more preferably by at least a factor 100, and even more preferably by at least a factor 1000, while the contribution to the detected signal by captured amplicon is (substantially) not suppressed.

As an example the case of a fluid cell having a height of 500 microns and containing labeled primers at a concentration of 1 micromolar is considered:
1) Without surface specific detection, the labeled primers give rise to a detected signal equivalent to ∼300000 labels per square micrometer. For a capture probe density of 10000 capture probes per square micrometer, in the best case this would result in a signal over background ratio of 1/30. For a typical hybridization experiment not all capture probes have bound to a labeled amplicon and the signal over background ratio will be even smaller; e.g., 1/300 for 1000 captured amplicons per square micrometer.
2) With surface specific detection, the labeled primers in solution give rise to a substantially lower detected signal; e.g., detected signal equivalent to ∼300 labeled primers per square micrometer for a background suppression factor of 1000. With surface-specific detection, the signal over background ratio is substantially improved to about 3 for 1000 captured labeled amplicons per square micrometer.

Another way to describe surface specific detection is to describe a surface-specific detector that detects those labels which are bound to the surface but does essentially not detect labels which are in solution. This means that the above definition given for surface specific detection applies equally to the term "a surface-specific detector that detects those labels which are bound to the surface but does essentially not detect labels which are in solution"

The capture probe molecule can be a DNA, RNA, PNA (peptide nucleic acid), LNA (locked nucleic acid), ANA (arabinonucleic acid), or HNA (hexitol nucleic acid) oligonucleotide. RNA, PNA, LNA, and HNA are able to form heteroduplexes with DNA that are more stable that DNA:DNA homoduplexes. This ensures enhanced discrimination ability for sequence mismatches (more specific hybridization). The higher stability of heteroduplexes also allows the use of shorter oligonucleotide probes at a given temperature reducing the chance of non-specific binding. PNA:DNA duplexes are formed independent of ionic strength of the hybridization buffer. This may enhance the hybridization efficiency in low salt PCR buffers.

It may be preferred according to the present invention to use capture probes with a higher melting temperature than the PCR primers and decreasing the temperature ramp down rate (as illustrated in figure 4). This means that the capture probes will anneal to the amplicons at a temperature which is above the annealing temperature for the primers. Thus, denatured amplicons are allowed to hybridize to the capture probes, before primer elongation and subsequent elongation can take place.

Hybridization of a portion of the amplicons means that the concentration of amplicon can be directly calculated from the intensity of the signal measured due to hybridization of amplicons to the capture probes. If the relation between the measured signal and amplicon concentration is not linear, a correction algorithm or calibration curve may be applied in order to deduce the amplicon concentration.

The capture portion of the capture probe may contain from 10 to 200 nucleotides, preferably from 15 to 50 nucleotides, more preferably from 20 to 35 nucleotides specific for the amplicons produced during the amplification reaction. The capture probe can also contain additional nucleotide sequences that can act as a spacer between the capture portion and the surface or that can have a stabilizing function that can vary from 0 to 200 nucleotides, preferably from 0 to 50. These non-capturing nucleotide sequences can either contain normal nucleotides or a-basic nucleotides.

The capture molecule may be immobilized by its 5' end, or by its 3' end.

For multiplexing, capture molecules are immobilized in specifically localized areas of a solid support in the form of a micro-array of at least 4 capture molecules per µm², preferably at least 1000 capture molecules per µm², more preferably at least 10000 capture molecules per µm², and even more preferably 100000 capture molecules per µm².

In a specific embodiment, the capture molecules comprise a capture portion of 10 to 100 nucleotides that is complementary to a specific sequence of the amplicons such that said capture potion defines two non-complementary ends of the amplicons and a spacer portion having at least 20 nucleotides and wherein the two non-complementary ends of the amplicons comprise a spacer end and a non-spacer end, respectively, such that the spacer end is non-complementary to the spacer portion of the capture molecule, and said spacer end exceeds said non-spacer end by at least 50 bases.

The terms "nucleic acid, oligonucleotide, array, nucleotide sequences, target nucleic acid, bind substantially, hybridizing specifically to, background, quantifying" are the ones described in the international patent application WO 97/27317 incorporated herein by reference. The term polynucleotide refers to nucleotide or nucleotide like sequences being usually composed of DNA or RNA sequences.

The terms "nucleotides triphosphate, nucleotide, primer sequence" are further those described in the documents WO 00/72018 and WO 01/31055 incorporated herein by references.

References to nucleotide(s), polynucleotide(s) and the like include analogous species wherein the sugar-phosphate backbone is modified and/or replaced, provided that its hybridization properties are not destroyed. By way of example, the backbone may be replaced by an equivalent synthetic peptide, called Peptide Nucleic Acid (PNA).

According to a preferred embodiment the capture probes are immobilized on the hybridization surface in a patterned array. According to a preferred embodiment the capture probes are immobilized on the surface of micro-arrays.

"Micro-array" means a support on which multiple capture molecules are immobilized in order to be able to bind to the given specific target molecule. The micro-array is preferentially composed of capture molecules present at specifically localized areas on the surface or within the support or on the substrate covering the support. A specifically localized area is the area of the surface which contains bound capture molecules specific for a determined target molecule. The specific localized area is either known by the method of building the micro-array or is defined during or after the detection. A spot is the area where specific target molecules are fixed on their capture molecules and can be visualized by the detector. In one particular application of this invention, micro-arrays of capture molecules are also provided on different or separate supports as long as the different supports contain specific capture molecules and may be distinguished form each other in order to be able to quantify the specific target molecules. This can be achieved by using a mixture of beads which have particular features and are able to be distinguishable from each other in order to quantify the bound molecules. One bead or a population of beads is then considered as a spot having a capture molecule specific to one target molecules.

Micro-arrays are preferentially obtained by deposition of the capture molecules on the substrate which is done by physical means such as pin or "pin and ring" touching the surface, or by release of a micro-droplet of solution by methods such as piezo- or nanodispenser.

Alternatively, in situ synthesis of capture molecules on the substrate of the embodiments of the inventions with light spatial resolution of the synthesis of oligonucleotides or polynucleotides in predefined locations such as provided by US 5,744,305 and US 6,346,413.

It may be preferred that the PCR mixture can be enriched for single stranded amplicons by means of asymmetrical PCR (or LATE PCR: linear after the exponential). In asymmetrical PCR, unequal concentrations of forward and reverse PCR primer are used. When the concentration of the labeled primer is higher that that of the unlabeled primer, the labeled strand will be amplified at a lower rate that the unlabeled strand. This not only leads to an accumulation of the labeled strand but also directly favors the hybridization of the labeled strand to the capture probe.

The term "real-time PCR" means a method which allows detection and/or quantification of the presence of the amplicons during the PCR cycles. In real-time PCR, the presence of the amplicons is detected and/or quantified in at least one of the amplification cycles. The increase of amplicons or signal related to the amount of amplicons formed during the PCR cycles are used for the detection and/or quantification of a given nucleotide sequence in the PCR solution. In a preferred embodiment, the presence of the amplicons is detected and/or quantified in every cycle.

The term "amplicon" in the invention related to the copy of the target nucleotide sequences being the product of enzymatic nucleic acid amplification

Instead of labeled PCR primers, internal labeling with labeled dNTPs can be used.

The label-associated detection methods are numerous. A review of the different labeling molecules is given in WO 97/27317. They are obtained using either already labeled primer, or by enzymatic incorporation of labeled nucleotides during the copy or amplification step (WO 97/27329) [intercalators are not preferred in this invention].

Possible labels are fluorochromes which are detected with high sensitivity with fluorescent detector. Fluorochromes include but are not limited tocyanin dyes (Cy3, Cy5 and Cy7) suitable for analyzing an array by using commercially available array scanners (as available from, for example, General Scanning, Genetic Microsystem). FAM (carboxy fluorescein) is also a possible alternative as a label. The person skilled in the art knows suitable labels which may be used in the context of this invention.

In a preferred embodiment of the invention, a signal increase of the fluorescence signal of the array related to the presence of the amplicons on the capture molecule is detected as compared to the fluorescence in solution.

In a particular embodiment the differences of the detection of the fluorophore present on the array is based on the difference in the anisotropy of the fluorescence being associated with a bound molecule hybridized on the capture molecule as a DNA double helix compared to the fluorescence being associated with a freely moving molecule in solution. The anisotropy depends on the mobility of the fluorophores and the lifetime of the fluorescence associated with the fluorophores to be detected. The method assay for the anisotropy on array is now available form Blueshift Biotechnologies Inc., 238 East Caribbean Drive, Sunnyvale, CA 94089 (http://www.blueshiftbiotech.com/dynamicfl.html).

In a particular embodiment, the detection of fluorophore molecules is obtained preferably in a timer-resolved manner. Fluorescent molecules have a fluorescent lifetime associated with the emission process. Typically lifetimes for small fluorophores such as fluorescein and rhodamine are in the 2-10 nanoscecond range. Time-resolved fluorescence (TRF) assays use a long-lived (>1000ns) fluorophore in order to discriminate assay signal from short-lived interference such as autofluorescence of the matrix or fluorescent samples which almost always have lifetimes of much less than 10 ns. Lifetime is preferably modulated by the nearby presence of another fluorophore or a quencher with which a resonant energy transfer occurs. Instruments for TRF simply delay the measurement of the emission until after the short-lived fluorescence has died out and the long-lived reporter fluorescence still persists. Fluorescence lifetime can be determined in two fundamental ways. The time domain technique uses very short pulses (picosecond) of excitation and then monitors the emission in real-time over the nanosecond lifetime. Fitting the decay curve to an exponential yields the lifetime. The frequency domain technique modulates the excitation at megahertz frequencies and then watches the emission intensity fluctuate in response. The phase delay and amplitude modulation can then be used to determine the lifetime. The frequency technique for fast and economical lifetime imaging is now available from Blueshift Biotechnologies Inc. As stated above, theses definitions apply to all of the described embodiments.

In one embodiment of the invention subject of the present invention is a method for simultaneous quantitative detection of multiple target nucleic acid sequences during amplification, wherein primers are labeled and the hybridized labeled amplicons are detected with a surface-specific detection system that detects those labels which are bound to the surface. Surface-specific means as defined above that labels which are in solution are essentially not detected.

In a preferred embodiment the signal of the label to be detected does not change in dependence of the binding state of said multiple nucleic acid sequences.

In an even more preferred embodiment the signal to be detected is a fluorescent signal. It is preferred an "always-on label" which are preferentially small organic fluorophores, but can also be a particulate label (either fluorescent or non-fluorescent), such as nano-phosphores, quantum dots.

For detection of multiple nucleic acids multiple labels may be used, in a preferred embodiment the same label is used for detection of each of said multiple target nucleic acid sequences.

This invention also describes a method for simultaneous quantitative analysis of multiple nucleic acid sequences in a single compartment by using e.g. an integrated nucleic acid microarray combined with a highly surface-specific readout device, the latter will be described below in detail.

The invention further relates to a device wherein a surface which is either part of the chamber surface or a surface that is created in the reaction chamber, such as a bead surface, is coated with capture probes and in the same chamber, a PCR reaction takes place.

Figure 1 describes a principle to monitor a PCR reaction with an in-tube microarray. The PCR mixture, containing template DNA and primers is applied to a containment of which (part of) the inner surface is coated with DNA capture probes (Figure 1A). When PCR thermocycling is started, specific segments of the template DNA will be amplified. Because one (or both) of the PCR primers is labeled with a fluorophore, the resulting PCR products (amplicons) will be fluorescently labeled (Figure 1B). As mentioned above instead of labeled primer labeled dNTPs can be used. Because (part of) the inner surface of the containment is coated with DNA capture probes that have a sequence that is complementary to a part of the amplicons, the amplicons can hybridize to the capture probes. Hybridization to the capture probes will only occur during the annealing phase of a PCR cycle. During the denaturation step of the PCR cycle, the hybridized amplicons will dissociate from the capture probes. During each cycle only a portion of the amplicons will hybridize. The majority of the amplicons will be elongated before they have diffused to the capture site. On the other hand, because preferentially a non-displacing DNA polymerase will be used, amplicons that are hybridized will not be amplified in that cycle.

Hybridized amplicons can be detected by an optical set-up with high surface-specificity. As the entire PCR volume is loaded with fluorescently labeled primers (or fluorescently labeled dNTPs), it is essential to use a surface-specific optical measurement that only detects fluorescence close to the hybridization surface (Figure 1C). The surface-specificity of the detector should be very high to achieve a good signal to background ratio (and as a consequence also good signal-to noise ratio).

During the annealing phase of each PCR cycle a fluorescence measurement should be conducted to measure the amount of fluorescently labeled amplicons that have hybridized to the capture surface. The amount of hybridized (and thus detectable) amplicons is representative of the amplicon concentration in the PCR mixture. The signal to cycle graph will be an S-curve. Similar to standard real-time PCR, the cycle number at which the signal reaches a certain intensity threshold is indicative of the initial target template concentration. This method can thus be used for quantitative detection of nucleic acid targets in a sample.

The surface-specific detection system is preferentially selected from a group comprising a confocal measurement device, a plasmonic measurement device and a device for the measurement according to evanescent detection.

As mentioned above, to be able to discriminate between hybridized amplicons and primers or amplicons in solution, it is essential to make a surface specific measurement. A surface specific measurement only detects labels that are very close to the capture surface. Because hybridization can only take place where capture probes have been deposited and the PCR mixture is homogeneous, it is possible subtract the background (the fluorescence intensity between the spots) and to determine the amount of hybridized amplicons.

Possible labels are fluorescent labels or non-fluorescent (e.g. particulate) where a difference in refractive index or absorption can be detected by optical means. It should be straightforward for someone skilled in the art to know suitable fluorescent or non-fluorescent labels.

For highly surface specific measurements, that are a suppression of the background by at least a factor 50, one can distinguish between three different approaches:
1. Confocal: typical measurement height along the optical axis of 1-2 µm.
2. Plasmonic: measurement height of about the wavelength of excitation light or smaller.
3. Evanescent: measurement height of 100 nm or smaller.

### 1. CONFOCAL

Confocal measurements can be made with various types of imaging equipment. Figure 3 shows a standard pinhole-based system. Such systems can be made very compact (PCT/IB2007/052499, PCT/IB2007/052634, and PCT/IB2007/052800). Different locations along the optical axis of the system give rise to different locations where the labels are imaged by the objective closest to the array surface. By using a pinhole and proper positioning -at the location where there is a sharp image of a label at the array surface- one can select a small measurement volume (depth along the optical axis of only 1-2 µm) in the neighborhood of the sensor surface.

### 2. PLASMONIC

Here the substrate is covered with a metal such as Au, Ag. The capture probes are on the metal layer or a spacing layer is deposited on top of the metal and subsequently covered with capture probes. The fluorescence of the labels of the hybridized DNA can couple to the surface plasmon at the metal medium/fluid interface. Labels in the fluid cannot couple or with a substantially smaller efficiency to the surface plasmon. By out coupling of the surface plasmon (that is converting the surface plasmon mode in to a propagating wave) and measuring the out coupled power, one essentially only measures the fluorescence of the labels of the hybridized DNA. As a result the fluorescence measurement is highly surface specific.

### 3. EVANESCENT

As another alternative method for enhancing the surface specificity, one can use evanescent excitation methods, where an evanescent wave is excited at the surface of the substrate and excites the fluorophores.

As a first method one can use total internal reflection (TIR) at the substrate-fluid interface, which results in measurement (excitation) volumes typically within 100-200 nm of the array surface. TIR however requires the use of a glass prism connected to the substrate or the use of a substrate with a wedge shape to enable the coupling of excitation light with angles above the critical angle of the substrate-fluid interface into the substrate.

Alternatively (as described in PCT/IB2006/051942, PCT/IB2006/054940) one can cover the substrate with a non-transparent medium such as a metal and pattern the metal with [an array of] apertures with at least one dimension in the plane parallel to the substrate-fluid interface below the diffraction limit of light in the fluid. As an example, one can pattern the substrate with wire grids that have one in-plane dimension above and the other dimension below the diffraction limit of the light in the fluid. This results in excitation volumes within 50 nm (Measurement volumes of 20-30 nm have already been demonstrated experimentally) of the array surface. Advantage of this method over the first method [for evanescent excitation] is that it is simpler- there is no need for a prism or wedge shaped surface and that there are no special requirements for the angle of incidence and shape of the excitation spot and one can use a simple CCD camera for imaging the fluorescence- and enables substantially smaller excitation volumes.

Subject of the present invention is further a device for conducting a method according to the present invention comprising a compartment of which an inner surface is coated with capture probes for multiple target nucleic acid sequences and a surface-specific detection system that detects those labels which are bound to the surface but does essentially not detect labels which are in solution.

Multiple different capture probes can be coated on the hybridization surface in a patterned array to simultaneously monitor the amplification of multiple different amplicons in the same fluid compartment. Figure 2 shows a schematic representation of an example of an array with 16 different capture spots. All capture spots monitor the amplification of a different amplicon within the same PCR mixture. This allows for much higher multiplex grades than currently possible. This allows for multiplex grades greater than 6 and up to 100 or more. An additional advantage of the embodiments of the invention is that only one fluorophore (one species) is needed which makes multiple expensive color filters and/or separated photodetectors unnecessary.

Thus, a device according to the present invention may be a micro-array.

In another embodiment of the invention the capture probes on the solid surface in the PCR compartment are folded probes (e.g. molecular beacons) or other probes (e.g. Taqman probes) with a fluorescent label and quencher in close proximity to one another due to the structure of the probe. In this embodiment the PCR reaction(s) is (are) performed without any label in the reaction or label attached to the amplification primers. Specific amplicons that are formed during the PCR reaction(s) can hybridize with the labeled capture probes on the solid surface, thereby either separating quencher and label (in case of molecular beacon type folded probes) or allowing the polymerase to hydrolyse the probe (in case of Taqman-like capture probes). As a result a fluorescent signal can be measured at the spot where the capture probes are located.

In this embodiment it is not a prerequisite to use a highly surface specific reader, since signal is only generated upon hybridization of amplicons to the capture probe. All other statements above also apply to this embodiment of the invention.

Thus, subject of the present invention is a method for simultaneous quantitative detection of multiple target nucleic acid sequences according to the present invention, wherein the capture probes are probes with a fluorescent label and a quencher in close proximity to one another due to the structure of the probes, and a signal may be detected in case an amplicon hybridizes to said capture probes.

A further subject of the present invention is a device for conducting a method according to the present invention comprising a compartment of which an inner surface is coated with capture probes for multiple target nucleic acid sequences and wherein the capture probes are labeled and a signal may be detected in case an amplicon hybridizes to said capture probes.

According to this embodiment of the device the label is preferentially selected from a group comprising molecular beacons, intercalating dyes, TaqMan probes.

Yet another embodiment of the present invention is a method for simultaneous quantitative detection of multiple target nucleic acid sequences, wherein the capture probes are immobilized on individually identifiable beads. In a preferred embodiment different beads have different capture probes.

It may be preferred that the beads are brought to the surface of the compartment and captured amplicons are measured by surface-specific detection. The beads may be brought to the surface e.g. by magnetic actuation.

In a preferred embodiment the fluorescence of a label is detected. As outlined above, a person skilled in the art knows further fluorescent and non-fluorescent labels.

If the capture probes are immobilized on beads, this allows for a quasi-homogeneous assay as the beads are freely dispersed in the reaction solution. Quasi-homogeneous assays allow for faster kinetics than non-homogeneous assays. The beads can have a diameter between 50 nm and 3 µm and are individually identifiable e.g. by color-coding, bar-coding or size. Multiple beads containing different capture probes may be present in the same reaction solution (Figure 5). Beads with captured amplicons on them, can be brought to the surface by magnetic actuation (when (para)magnetic beads are used) or by dielectrophoresis (when non-magnetic beads are used). On the surface the beads can be identified and the captured amplicons can be detected by a surface-specific optical measurement.

Another method to distinguish different beads is based on the differences in resonance wavelength of the resonator modes propagating along the perimeter of the bead due to size differences. In this case the bead acts as a resonator that supports resonator modes propagating along the perimeter of the bead (for a spherical bead these resonator modes are so-called whispering gallery modes). The resonator is in-resonance for a wavelength where the phase shift after one roundtrip along the perimeter is a multiple of 2*pi. These resonator modes also have an evanescent field that extends into the environment (fluid) of the bead. A fluorophore in the near field region (typically <100 nm) of the bead can couple a fraction of it's fluorescence to the resonator modes supported by the bead. The fraction coupled to the resonator mode is stronger for on-resonance wavelengths than for other wavelengths and this results in a modulation of the fluorescence spectrum with the resonance peaks corresponding to the resonator modes or whispering gallery modes. The typical diameter of the beads is larger than 1 µm up to 50 µm. Detection can be performed throughout the whole volume, because the fluorescence due to fluorophores that are not bound to the bead have the intrinsic spectrum of the fluorophore, and there is no need for a surface-specific optical measurement.

Thus, subject of the invention is also a device for conducting a method according to the invention comprising a compartment with individually identifiable beads wherein capture probes for multiple target nucleic acid sequences are immobilized on said individually identifiable beads.

In a preferred embodiment the device further comprises means for bringing the beads to the surface of the compartment and a surface-specific detector that detects those labels which are bound to the surface but does essentially not detect labels which are in solution.

## Claims

1. Method for simultaneous real-time quantitative detection of multiple target nucleic acid sequences during amplification,
wherein the number of simultaneously and quantitatively detected target nucleic acids that are amplified and detected in a single compartment is greater than six, preferably greater than seven, more preferably greater than ten.

2. Method for simultaneous real-time quantitative detection of multiple target nucleic acid sequences during amplification,
wherein surface-immobilized oligonucleotide probes complementary to said multiple nucleic acid sequences act as capture probes for said multiple amplified nucleic acid sequences,
and wherein detection of said multiple amplified nucleic acid sequences captured to the captured sites is performed with a surface-specific detection method detecting a signal in proximity to the surface.

3. Method for simultaneous real-time quantitative detection of multiple target nucleic acid sequences during amplification according to claim 2 wherein surface specific detection is obtained either by a surface specific detection system and/or by surface-specific generation of the signal to be detected.

4. Method for simultaneous quantitative detection of multiple target nucleic acid sequences during amplification according to claims 1 or 3 comprising the following steps:
- Providing a device having a reaction compartment, wherein the reaction compartment comprises a surface that is coated with multiple capture probes for said multiple nucleic acid sequences,
- adding multiple target nucleic acids and an amplification mixture comprising amplification primer for the amplification of said multiple target nucleic acids to the compartment,
- starting amplification thermocycling during which specific segments of said target nucleic acids are amplified thereby creating amplicons,
wherein during the annealing phase of the amplification reaction a reproducible portion of the amplicons hybridize to the capture probes, and
wherein amplicons which are hybridized to said capture probes are specifically and quantitatively detected, wherein the respective signal which is detected is indicative of the initial respective target nucleic acid concentration and thereby the multiple target nucleic acid sequences are quantitatively detected.

5. Method for simultaneous quantitative detection of multiple target nucleic acid sequences during amplification according to claims 1 to 4, wherein primers are labelled and the hybridized labeled amplicons are detected with a surface-specific detection system.

6. Method for simultaneous quantitative detection of multiple target nucleic acid sequences during amplification according to claim 5, wherein the signal of the label to be detected does not change in dependence of the binding state of said multiple nucleic acid sequences.

7. Method for simultaneous quantitative detection of multiple target nucleic acid sequences during amplification according to claims 2 to 6, wherein the capture probes are immobilized on the hybridization surface in a patterned array.

8. Method for simultaneous quantitative detection of multiple target nucleic acid sequences according to claim1 to 7, wherein the same label is used for detection of each of said multiple target nucleic acid sequences.

9. Method for simultaneous quantitative detection of multiple target nucleic acid sequences according to claims 1 to 4, wherein the capture probes are probes with a fluorescent label and a quencher in close proximity due to the structure of the probes, and a signal may be detected in case an amplicon hybridizes to said capture probes.

10. Method for simultaneous quantitative detection of multiple target nucleic acid sequences according to claims 1 to 4, wherein the capture probes are probes with a fluorescent label and a quencher, and a signal may be detected in case a target nucleic acid sequence hybridized to said capture probes causing enzymatic hydrolysis of the capture probes thereby liberating said quencher from said fluorophore.

11. Method for simultaneous quantitative detection of multiple target nucleic acid sequences according to claims 1 to 4, wherein the capture probes are immobilized on individually identifiable beads.

12. Method for simultaneous quantitative detection of multiple target nucleic acid sequences according to claim 11, wherein different beads have different capture probes.

13. Method for simultaneous quantitative detection of multiple target nucleic acid sequences according to claim 11 or 12, wherein the beads are brought to the surface of the compartment and captured amplicons are measured by surface-specific detection.

14. A device for conducting a method according to claims 1 to 9 comprising a compartment of which an inner surface is coated with capture probes for multiple target nucleic acid sequences and a surface-specific detection system that detects those label which are bound to the surface but does essentially not detect labels which are in solution.

15. A device for conducting a method according to claims 1 to 4, 9 and 10 comprising a compartment of which an inner surface is coated with capture probes for multiple target nucleic acid sequences and wherein the capture probes are labeled and a signal may be detected in case an amplicon hybridizes to said capture probes.

16. A device according to claim 15, wherein the capture probes are probes with a fluorescent label and a quencher, and a signal may be detected in case a target nucleic acid sequence hybridized to said capture probes causing enzymatic hydrolysis of the capture probes thereby liberating said quencher from said fluorophore.

17. A device for conducting a method according to claims 1 to 4, and 11 to 14 comprising a compartment with individually identifiable beads wherein capture probes for multiple target nucleic acid sequences are immobilized on said individually identifiable beads.

18. A device according to claim 17, wherein the device further comprises means for bringing the beads to the surface of the compartment and a surface-specific detector that detects those labels which are bound to the surface but does essentially not detect labels which are in solution.

19. Use of a device according to claims 14 to 18 for simultaneous quantitative analysis of multiple target nucleic acid sequences.

20. Use of a surface-specific detection method detecting a signal in proximity to the surface for real-time PCR.

21. Use of a surface-specific detection method for real-time PCR according to claim 20, wherein surface specific detection is obtained either by a surface specific detection system and/or by surface-specific generation of the signal to be detected.
